(19) Europäisches Patentamt
European Patent Office
Office européen des brevets

(11) Publication number: **0 443 848 B1**

(12) **EUROPEAN PATENT SPECIFICATION**

(45) Date of publication of patent specification: **21.09.94**

(51) Int. Cl.[5]: **C07C 49/717**, A61K 31/12

(21) Application number: **91301377.7**

(22) Date of filing: **21.02.91**

(54) **Novel anti-ulcer substance.**

(30) Priority: **22.02.90 JP 39663/90**
**29.11.90 JP 325333/90**

(43) Date of publication of application:
**28.08.91 Bulletin 91/35**

(45) Publication of the grant of the patent:
**21.09.94 Bulletin 94/38**

(84) Designated Contracting States:
**AT BE CH DE DK ES FR GB GR IT LI LU NL SE**

(56) References cited:
**US-A- 4 749 681**

**CHEMICAL ABSTRACTS, vol. 101, no. 3, July 16, 1984, Columbus, Ohio, USA; H. ORSZANSKA et al, "Lactone synthesis in the 1,1,4-trimethyl cycloheptane series via the Wittig-Horner reaction" page 580, column 1, abstract-no. 23246c**

**PATENT ABSTRACTS OF JAPAN, unexamined applications, C field, vol. 10, no. 287, September 30, 1986, THE PATENT OFFICE JAPANESE GOVERNMENT, page 127 C 375**

**The Merck Index, eleventh edition (1989), p. 310, item 2017.**

**The Pharmacopoeia of Japan, eleventh edition (1986), English version, pp. 1116-1117.**

(73) Proprietor: **TOKYO TANABE COMPANY LIMITED**
**2-6, Nihonbashi-Honcho 2-chome**
**Chuo-ku Tokyo (JP)**

(72) Inventor: **Murakami, Kiyokazu**
**3-22, Nakatehara 2-chome,**
**Kouhoku-ku**
**Yokohama-shi, Kanagawa (JP)**
Inventor: **Yokura, Susumu**
**2221-6, Matoba**
**Kawagoe-shi, Saitama (JP)**

(74) Representative: **Holmes, Michael John et al**
**Frank B. Dehn & Co.**
**Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 443 848 B1

## Description

This invention relates to an antiulcer agent containing, as an effective ingredient, 4,5-dihydroxy-2,6,6,-trimethyl-2-cyclohepten-1-one.

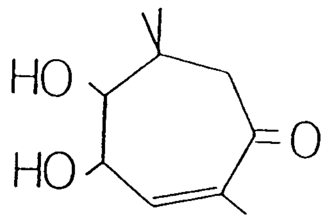

(hereinafter referred to as "Saishin N") obtainable from a crude drug known as "Saishin" (asiasari radix).

"Saishin" is a crude drug described in the Japanese Pharmacopoeia as a root and rootstock of "Usubasaishin" (Asiasarum sieboldii F. Maekawa) or "Keirinsaishin" (Asiasarum heterotropoides F. Maekawa var. mandshuricum F. Maekawa) which are members of the Aristolochiaceae, stripped of as much of the overground portion as possible. Saishin is prescribed as an antitussive expectorant or anodyne. As pharmacological effects of Saishin, antiallergic action, cardiac action, sedative action, antipyretic and analgesic actions, and anti-inflammatory action are known, but there has as yet been no report of antiulcer action.

The present inventors have for some time studied the ingredient composition of crude drugs and have now found that one of the previously unidentified components of Saishin shows antiulcer activity. This has been termed Saishin N. The present invention is based on this finding.

Other anti-ulcer drugs described in the prior art include Cetraxate (The Merck Index, 11th Edition, 1989, p310).

According to the present invention, there is provided in one aspect use of 4,5-dihydroxy-2,6,6-trimethyl-2-cyclohepten-1-one and/or the individual stereoisomers thereof for the preparation of a composition for use in the treatment of ulcers.

Further aspects provide 4,5-dihydroxy-2,6,6-trimethyl-2-cyclohepten-1-one and/or the individual stereoisomers thereof (Saishin N) and an antiulcer agent containing Saishin N as an active ingredient.

The Saishin N to be used in the present invention can be produced by extracting Saishin with an organic solvent for example chloroform, followed by fractionation to purify the extract e.g. by column chromatography for example on silica gel using a suitable mixture of solvents as eluent in an e.g. stepwise elution. The mixed solvent can be made up from hexane, chloroform, ethyl acetate, benzene and acetone.

A still further aspect of the invention therefore provides a process for the preparation of 4,5-dihydroxy-2,6,6-trimethyl-2-cyclohepten-1-one and/or the individual stereoisomers thereof which comprises extracting Saishin with an organic solvent, followed by fractionation.

Saishin N can also be synthesized chemically from eucarvone (2,6,6-trimethylcyclohepta-2,4-dien-1-one).

A yet further aspect of the invention therefore provides a process for the preparation of 4,5-dihydroxy-2,6,6-trimethyl-2-cyclohepten-1-one and/or the individual stereoisomers thereof which comprises subjecting 4,5-epoxy-2,6,6-trimethylcyclopenta-2-en-1-one to epoxide ring opening reaction.

Ring opening of the epoxy group may be effected by treating with an acid. 4,5-epoxy-2,6,6,-trimethylcyclopenta-2-en-1-one may be prepared by oxidation of eucarvone. Suitable oxidizing agents include organic peracids such as m-chloroperbenzoic acid, perbenzoic acid and permaleic acid; inorganic peracids such as aqueous hydrogen peroxide, perchloric acid, perbromic acid and periodic acid; and oxygen. Suitable acids for ring opening include inorganic acids such as perchloric acid, perbromic acid, periodic acid, hydrochloric acid, sulfuric acid and nitric acid; and organic acids such as formic acid, acetic acid, propionic acid, butyric acid, p-toluenesulfonic acid and methanesulfonic acid.

In the present invention, Saishin N which is extracted from other crude drugs or synthesized by the other synthesis methods may of course also be used.

In the following, inhibitory effects of Saishin N on four kinds of experimental ulcers are described in detail. Each experiment was carried out by using male Wistar rats weighing about 200 g in groups of six, and Saishin N was used by suspending in a 0.5 % sodium carboxymethyl cellulose solution. Cetraxate hydrochloride used as a control was also used by dissolving or suspending in the same solution. Activities against respective ulcers were evaluated by an inhibiting ratio obtained by dividing the difference between

the ulceration indexes of the non-administered group and the Saishin N group by the non-administered group.

⟨Hydrochloric acid-Ethanol-Induced Erosions⟩

To each rat which had been fasted for 24 hours was orally administered 0.5 ml of a mixed solution of 150 mM hydrochloric acid and 60 % ethanol per 100 g of weight. Each rat was slaughtered after one hour, a length of ulcer formed at the fundus gland area of stomach was measured and ulceration index was calculated based thereon. Cetraxate hydrochloride which is a control and Saishin N were each orally administered 30 minutes before administration of a hydrochloride-ethanol mixed solution.

⟨Aspirin-Induced Erosions⟩

In each rat which had been fasted 24 hours, the pyloric end of the stomach was ligated, and simultaneously Saishin N and cetraxate hydrochloride as a control were each administered into duodenum and then 150 mg/kg of aspirin was orally administered after 5 minutes, respectively. After 9 hours from ligation, each rat was slaughtered, a length of ulcer formed at the fundus gland area of stomach was measured and ulceration index was calculated based thereon.

⟨Water-immersion stress-Induced Erosions⟩

Each rat which had been fasted for 15 hours was immobilized in a stress cage and immersed chest-deep in a water bath at 21 °C. Each rat was slaughtered after 10 hours, a length of ulcer formed at the fundus gland area of stomach was measured and ulceration index was calculated based thereon. Cetraxate hydrochloride as a control and Saishin N were each orally administered 10 minutes before exposure to stress.

⟨Shay's Ulcer⟩

In each rat which had been fasted 48 hours, the pyloric end of the stomach was ligated, and each was kept without giving any food or water for 14 hours. Subsequently, each rat was slaughtered, and area of ulcer formed in the forestomach was measured and ulceration index was calculated based thereon. Cetraxate hydrochloride as a control and Saishin N were each administered into duodenum immediately after ligation.

The results are shown in Table 1.

Table 1

| | Dose mg/kg | Hydro-chloric acid-ethanol | Aspirin | Water-immersion stress | Shay |
|---|---|---|---|---|---|
| Saishin N | 20 | 88** | --- | --- | -- |
| | 50 | 99** | 52** | 49** | -- |
| | 100 | --- | 60** | 62** | 49** |
| | 300 | --- | --- | --- | 58** |
| Cetraxate hydro-chloride | 300 | 86** | 33* | 45* | 11 |

*; P < 0.05
**; P < 0.01

As can be clearly see from Table 1, Saishin N has an excellent antiulcer activity.

An effect on gastric acid secretion of Saishin N will now be described in detail.

The Gastric acid secretion inhibitory effect of Saishin N was tested with male Wistar rats, weighing about 200 g, in groups of six. In each rat which had been fasted 24 hours, the pyloric end of the stomach was ligated, 10 to 100 mg/kg of Saishin N was administered, and total acidity of gastric juice of each rat was measured after 4 hours. Saishin N was administered in to the duodenum immediately after ligation by suspending in a 0.5 % sodium carboxymethyl cellulose aqueous solution. Gastric juice was collected by slaughtering each rat and laparotomizing. Total acidity of the gastric juice was measured by titrating the gastric juice with a 0.1 N sodium hydroxide aqueous solution until a pH of the gastric juice reaches 7.0. As a control experiment, total acidity of the gastric juice of an untreated group was also measured in the same manner as described above.

The results are shown in Table 2.

Table 2

| | Dose mg/kg | Gastric acid secretion ml | Titrated 0.1 N NaOH ml | Total acidity μEq/4 hr | pH |
|---|---|---|---|---|---|
| Control | | 6.6 | 1.00 | 660 | 1.32 |
| Saishin N | 50 | 7.0 | 0.96 | 672 | 1.30 |
| | 100 | 6.5 | 0.95 | 618 | 1.31 |

As clearly seen from Table 2, Saishin N does not show any gastric acid secretion-inhibitory activity. Accordingly, the antiulcer effect of Saishin N is related to its cytoprotective activity.

(Acute toxicity)

Using 6-weeks old male SD rats, the minimum lethal dose (MLD) of Saishin N was examined. The results showed that the MLD value was 1000 mg/kg or more for oral administration.

When considering the above experimental results, it can be said that a drug containing Saishin N as an effective ingredient is an excellent antiulcer agent. The dose of Saishin N to a patient may vary depending on age, conditions, etc., but is generally 1 to 1000 mg, preferably 10 to 600 mg per day for an adult in oral administration, and it is preferably administered by dividing into 1 to 6 times, more preferably 1 to 3 times.

In the present invention, by admixing conventional pharmaceutical carriers to Saishin N, it can be formed into solid preparations such as tablets, hard or soft capsules, granules, powder, fine particles or suppository; or into liquid preparations such as injection, syrups, elixirs, suspensions or emulsions, etc. Solid preparations may be prepared in the form of enteric coated preparations or gradually releasing preparations. As a carrier used for these preparations, there may be optionally selected depending on the desired type of preparations such as excipients, binders, disintegrants, lubricants, coating agents, dissolving adjuvants, emulsifiers, suspending agents, surfactants, absorption adjuvants, stabilizers or solvents, etc. Examples of carriers include starches, dextrins, α, β or γ-cyclodextrin, glucose, lactose, sucrose, mannitol, sorbitol, partially alpharized starch, methyl cellulose, ethyl cellulose, hydroxyethyl cellulose, carboxymethyl cellulose, calcium carboxymethyl cellulose, sodium cross-linked carboxymethyl cellulose, crystal cellulose, low-substitution degree hydroxypropyl cellulose, calcium stearate, magnesium stearate, sodium arginate, magnesium silicate, calcium hydrogen phosphate, calcium carbonate, magnesium carbonate, magnesium metasilicic aluminate, Witepsol W35, Witepsol E85, Witepsol H15, polyvinyl alcohol, silicic anhydride, synthesized aluminium silicate, titanium oxide, talc, waxes, hydroxypropyl cellulose, hydroxypropylmethyl cellulose, hydroxyethylmethyl cellulose, carboxymethylethyl cellulose, cellulose acetate phthalate, cellulose acetate maleate, hydroxypropylmethyl cellulose phthalate, hydroxypropylmethyl cellulose acetate succinate, polyvinylalcohol phthalate, styrene-maleic anhydride copolymer, polyvinylacetal diethylaminoacetate, methacrylic acid-methyl methacrylate copolymer, methacrylic acid-ethyl acrylate copolymer, methacrylic acid-butyl methacrylate and dimethylaminoethyl methacrylate copolymer, ethyl acrylatemethyl methacrylate and ammonium trimethyl chlorideethyl methacrylate copolymer, gelatin, glycerin, propylene glycol, sodium lauryl sulfate, medium chain aliphatic acid triglyceride, lecitin, sorbitan monooleate, sorbitan sesquioleate, polyoxyethylene sorbitan monooleate, glyceryl monostearate, glyceryl monooleate, polyoxyethylene cetyl ether, polyoxyethylene cured castor oil, cane sugar aliphatic acid ester, polyglycerin aliphatic acid ester, polyoxyethylene-polyoxypropylene block copolymer, polyethylene glycol, polyvinyl pyrrolidone, cross-linked polyvinyl pyrrolidone or oil and fats such as cocoa butter, laurin butter, and glycerogelatin, etc.

The present invention will now be explained in more detail by referrence to the following non-limiting examples of preparation methods for Saishin N and examples of compositions.

Method example 1:

1500 g of Saishin was pulverized by an atomizer, and extracted with 4 liters of chloroform under heat-refluxing for one hour. This extraction step was repeated three times, and the extracts were combined and condensed under reduced pressure to obtain 93 g of an extract. Next, the extract was subjected to contact treatment by 2 liters of n-hexane three times in total to obtain 87 g of n-hexane-soluble extract. The extract was applied to column chromatography using 800 g of silica gel, and succesively eluted by using a mixed solution of n-hexane : ethyl acetate while changing the mixing ratio thereof (volume ratio = 50 : 1 - 10 : 1). Then, eluted portion by chloroform was collected and condensed under reduced pressure to obtain 4.48 g of crude product. This crude product was applied to column chromatography using 70 g of silica gel and eluted by using a mixed solution of benzene : acetone (volume ratio = 60 : 1) successively. Then, eluted portion by benzene : acetone (volume ratio = 10 : 1) was collected and condensed under reduced pressure to obtain 730 mg of crude product. This was dotted on a silica gel thin layer plate for preparation with 30 mg per a plate and developed three times with benzene : acetone (volume ratio = 9 : 1) and then developed once with a mixed solution of n-hexane : ethyl acetate : ethanol (volume ratio = 4 : 1 : 1). Subsequently, the Saishin N portion, as judged from UV absorption and coloring by anisaldehyde sulphuric reagent was scraped off and extracted with chloroform to obtain 72 mg of Saishin N.
IR Spectrum (KBr, $cm^{-1}$): 3450, 2980, 2950, 1680, 1450, 1370.
$^1$H-Nuclear magnetic Resonance Spectrum ($CDCl_3$, ppm):
6.50 (1H, m), 4.35 (1H, m), 3.22 (1H, d), 2.52 (1H, d), 2.34 (1H, d), 1.83 (3H, dd), 1.11 (3H. s), 1.01 (3H, S).
$^{13}$C-Nuclear magnetic Resonance Spectrum ($CDCl_3$, ppm) :
19.09 (q), 22.86 (q), 27.73 (q), 34.50 (s), 54.54 (t), 69.94 (d), 81.15 (d), 138.34 (s), 145.36 (d), 201,57 (s).

Method example 2:

4,5-Epoxy-2,6,6,-trimethylcyclopenta-2-en-1-one

To a methylene chloride (20 ml) solution containing eucarvone (1.5 g, 10 mmole) dissolved therein was added m-chloroperbenzoic acid (2.37 g, 11 mmole) over 10 minutes at room temperature under stirring, and the mixture was stirred at room temperature for one hour. Precipitates were removed by filtration, and the filtrate was diluted with hexane, washed with a dil. aqueous sodium hydroxide solution and a saturated saline solution, dried over magnesium sulfate, filtered and then condensed. The residue after condensation was purified by silica gel column chromatography using hexane-isopropyl ether (5 : 1) as an eluent to obtain 1.56 g (Yield: 93 %) of the colorless oily product of the headline.
$^1$H-Nuclear magnetic Resonance Spectrum ($CDCl_3$, ppm):
1.03 (3H, s), 1.27 (3H, s), 1.90 (3H, d J=2Hz), 2.21 (1H, dd, J=13,2Hz), 2.89 (1H, d, J=13Hz), 3.14 (1H, dd, J=4, 2Hz), 3.34 (1H, dd, J=6, 4Hz), 6.48 (1H, dq, J=6, 2Hz)

Saishin N

Then, to a tetrahydrofuran (10 ml) solution containing the above epoxide (0.97 g, 5.8 mmole) dissolved therein was added a 10 % perchloric acid (2 ml) under ice-cooling and stirring, and the mixture was stirred for 6 hours, followed by stirring at room temperature overnight. The reaction mixture was diluted by ethyl acetate, washed with a dil. aqueous sodium hydroxide solution and a saturated saline solution, dried over sodium sulfate, filtered and then condensed. The residue after condensation was purified by silica gel column chromatography using benzene-ethyl acetate (3 : 1) as an eluent, followed by crystallization from a mixed solution of benzene and hexane to obtain 598 mg (Yield: 56 %) of Saishin N as a colorless plate crystal (melting point; 77.5 to 78.5 °C).
$^1$H-Nuclear magnetic Resonance Spectrum ($CDCl_3$, ppm):
1.01 (3H, s), 1.11 (3H, s), 1.83 (3H, dd, J=2.0, 1.5Hz), 2.35 (1H, d, J=12Hz), 2.52 (1H, d, J=12Hz), 3.22 (1H, d, J=9Hz), 4.35 (1H, d.quintet, J=9, 2.0Hz), 6.50 (1H, m).

Composition example 1 (Tablets):

| | weight (%) |
|---|---|
| (1) Saishin N | 30.0 |
| (2) Silicic anhydride | 20.0 |
| (3) Lactose | 10.0 |
| (4) Calcium carboxymethyl cellulose | 10.0 |
| (5) Hydroxypropyl cellulose | 3.0 |
| (6) Polyoxyethylene (40) monostearate | 0.5 |
| (7) Crystalline cellulose | 26.0 |
| (8) Magnesium stearate | 0.5 |
| | 100.0 |

The above (1) and (2) were mixed, and after adding (3) to (5) to them, they were mixed. To the mixture was added an aqueous solution containing (6) dissolved therein, and the mixture was granulated and then dried. The resulting granules were regulated in particle size, (7) and (8) were further added to effect mixing and formulated under pressure to prepare tablets each having 250 mg.

Composition example 2 (Hard capsules):

| | weight (%) |
|---|---|
| (1) Saishin N | 20.0 |
| (2) Silicic anhydride | 15.0 |
| (3) Corn starch | 20.0 |
| (4) Lactose | 40.5 |
| (5) Polyoxyethylene (20) sorbitan monooleate | 0.5 |
| (6) Hydroxypropyl cellulose | 3.0 |
| (7) Magnesium stearate | 1.0 |
| | 100.0 |

The above (1) and (2) were mixed and after adding (3) , (4) and (6) to them, they were mixed. To the mixture was added and mixed ethanol containing (5) dissolved therein, and the mixture was dried to prepare granules. To the granules was added (7) and mixed, they were filled in a hard capsule to prepare hard capsules each containing 300 mg.

Composition example 3 (Granules):

| | weight (%) |
|---|---|
| (1) Saishin N | 10.0 |
| (2) Silicic anhydride | 7.5 |
| (3) Lactose | 69.5 |
| (4) Low substitution degree hydroxypropyl cellulose | 10.0 |
| (5) Polyvinylpyrrolidone | 2.0 |
| (6) Lauryl sodium sulfate | 1.0 |
| | 100.0 |

The above (1) and (2) were mixed, and after adding (3) to (5) to them, they were mixed. To the mixture was added a solution containing (6) dissolved therein, and the mixture was kneaded and granulated by using an extrusion granulating machine to prepare cylindrical granules.

Composition example 4 (Soft capsules):

| | weight (%) |
|---|---|
| (1) Saishin N | 25.0 |
| (2) Polyethylene glycol 400 | 67.0 |
| (3) Polyoxyethylene (20) sorbitan monooleate | 5.0 |
| (4) Purified water | 3.0 |
| | 100.0 |

The above (1) to (4) were thoroughly mixed under heating, and the dispersion obtained was filled in a soft capsule by the conventional method to prepare soft capsules each containing 400 mg.

Composition example 5 (Suppository):

| | weight (%) |
|---|---|
| (1) Saishin N | 5.0 |

| | |
|---|---|
| (2) Wiptesol H15 | 85.0 |
| (3) Lauric acid triglyceride | 9.0 |
| (4) Lecitin | 1.0 |
| | 100.0 |

The above (1) to (4) were thoroughly mixed under heating, and cooled to solidify them. It was melted at 50 to 60 °C and after cooling to 32 to 35 °C, injected to a suppository mold, allowed to stand for cooling and solidified to prepare suppositories each having 2 g.

**Claims**

**Claims for the following Contracting States : DE, GB, FR, IT, NL, SE, LI, CH, BE, AT, LU, DK**

1. Use of 4,5-dihydroxy-2,6,6-trimethyl-2-cyclohepten-1-one or the individual stereoisomers thereof for the preparation of a composition for use in the treatment of ulcers.

2. 4,5-dihydroxy-2,6,6-trimethyl-2-cyclohepten-1-one and/or the individual stereoisomers thereof.

3. 4,5-dihydroxy-2,6,6-trimethyl-2-cyclohepten-1-one and/or the individual stereoisomers thereof as claimed in claim 2 in substantially pure form.

4. 4,5-dihydroxy-2,6,6-trimethyl-2-cyclohepten-1-one and/or the individual stereoisomers thereof as claimed in claim 2 in crystalline form.

5. 4,5-dihydroxy-2,6,6-trimethyl-2-cyclohepten-1-one and/or the individual stereoisomers thereof as claimed in claim 2 substantially free from other material of the root and rootstock of Asiasarum (Asiasari Radix).

6. 4,5-dihydroxy-2,6,6-trimethyl-2-cyclohepten-1-one and/or the individual stereoisomers thereof as claimed in any one of claims 2 to 5 for use in the treatment of ulcers.

7. A process for the preparation of 4,5-dihydroxy-2,6,6-trimethyl-2-cyclohepten-1-one and/or the individual stereoisomers thereof which comprises extracting the root and rootstock of Asiasarum (Asiasari Radix) with an organic solvent, followed by fractionation.

8. A process as claimed in claim 7 wherein the root and rootstock of Asiasarum (Asiasari Radix) is extracted at least once with chloroform.

9. A process as claimed in claim 7 or claim 8 wherein the fractionation comprises column chromatography on silica gel using a mixed solvent eluent made up from two or more solvents selected from hexane, chloroform, ethyl acetate, benzene and acetone.

10. A process for the preparation of 4,5-dihydroxy-2,6,6-trimethyl-2-cyclohepten-1-one and/or the individual stereoisomers thereof which comprises subjecting 4,5-epoxy-2,6,6-trimethylcyclopenta-2-en-1-one to epoxide ring opening reaction.

11. A process as claimed in claim 10 wherein 4,5-epoxy-2,6,6,-trimethylcyclopenta-2-en-1-one is prepared by oxidising 2,6,6-trimethylcyclohepta-2,4-dien-1-one.

12. A process as claimed in claim 10 or claim 11 wherein the epoxide ring opening reaction is effected by treating 4,5-epoxy-2,6,6-trimethylcyclopenta-2-en-1-one with an acid.

13. A process as claimed in claim 11 or claim 12 wherein 2,6,6,-trimethylcyclohepta-2,4-dien-1-one is oxidised using an oxidant selected from organic or inorganic peracids and oxygen.

**14.** A pharmaceutical composition for use in the treatment of ulcers comprising 4,5-dihydroxy-2,6,6-trimethyl-2-cyclohepten-1-one and/or the individual stereoisomers thereof together with at least one pharmaceutically acceptable carrier or excipient.

**Claims for the following Contracting States : ES, GR**

**1.** Use of 4,5-dihydroxy-2,6,6-trimethyl-2-cyclohepten-1-one or the individual stereoisomers thereof for the preparation of a composition for use in the treatment of ulcers.

**2.** A process for the preparation of 4,5-dihydroxy-2,6,6-trimethyl-2-cyclohepten-1-one and/or the individual stereoisomers thereof which comprises extracting the root and rootstock of Asiasarum (Asiasari Radix) with an organic solvent, followed by fractionation.

**3.** A process as claimed in claim 2 wherein the root and rootstock of Asiasarum (Asiasari Radix) is extracted at least once with chloroform.

**4.** A process as claimed in claim 2 or claim 3 wherein the fractionation comprises column chromatography on silica gel using a mixed solvent eluent made up from two or more solvents selected from hexane, chloroform, ethyl acetate, benzene and acetone.

**5.** A process for the preparation of 4,5-dihydroxy-2,6,6-trimethyl-2-cyclohepten-1-one and/or the individual stereoisomers thereof which comprises subjecting 4,5-epoxy-2,6,6-trimethylcyclopenta-2-en-1-one to epoxide ring opening reaction.

**6.** A process as claimed in claim 5 wherein 4,5-epoxy-2,6,6,-trimethylcyclopenta-2-en-1-one is prepared by oxidising 2,6,6-trimethylcyclohepta-2,4-dien-1-one.

**7.** A process as claimed in claim 5 or claim 6 wherein the epoxide ring opening reaction is effected by treating 4,5-epoxy-2,6,6-trimethylcyclopenta-2-en-1-one with an acid.

**8.** A process as claimed in claim 6 or claim 7 wherein 2,6,6,-trimethylcyclohepta-2,4-dien-1-one is oxidised using an oxidant selected from organic or inorganic peracids and oxygen.

**Patentansprüche**

**Patentansprüche für folgende Vertragsstaaten : DE, GB, FR, IT, NL, SE, LI, CH, BE, AT, LU, DK**

**1.** Verwendung von 4,5-Dihydroxy-2,6,6-trimethyl-2-cyclohepten-1-on oder seiner einzelnen Stereoisomere zur Herstellung einer Zusammensetzung zur Verwendung bei der Behandlung von Magengeschwüren.

**2.** 4,5-Dihydroxy-2,6,6-trimethyl-2-cyclohepten-1-on beziehungsweise seine einzelnen Stereoisomere.

**3.** 4,5-Dihydroxy-2,6,6-trimethyl-2-cyclohepten-1-on beziehungsweise seine einzelnen Stereoisomere nach Anspruch 2 in einer im wesentlichen reinen Form.

**4.** 4,5-Dihydroxy-2,6,6-trimethyl-2-cyclohepten-1-on beziehungsweise seine einzelnen Stereoisomere nach Anspruch 2 in kristalliner Form.

**5.** 4,5-Dihydroxy-2,6,6-trimethyl-2-cyclohepten-1-on beziehungsweise seine einzelnen Stereoisomere nach Anspruch 2, im wesentlichen frei von anderem Material der Wurzel und dem Wurzelstock des Asiasarum (Asiasari Radix).

**6.** 4,5-Dihydroxy-2,6,6-trimethyl-2-cyclohepten-1-on beziehungsweise seine einzelnen Stereoisomere nach jedem der Ansprüche 2 bis 5 zur Verwendung bei der Behandlung von Magengeschwüren.

**7.** Verfahren zur Herstellung von 4,5-Dihydroxy-2,6,6-trimethyl-2-cyclohepten-1-on beziehungsweise seiner einzelnen Stereoisomere, das das Extrahieren der Wurzel und des Wurzelstocks des Asiasarum (Asiasari Radix) durch ein organisches Lösungsmittel enthält, gefolgt von Fraktionierung.

**8.** Verfahren nach Anspruch 7, wobei die Wurzel und der Wurzelstock des Asiasarum (Asiasari Radix) mindestens einmal mit Chloroform extrahiert wird.

**9.** Verfahren nach Anspruch 7 oder Anspruch 8, wobei die Fraktionierung, bei der ein gemischtes Lösungseluens verwendet wird, eine Säulenchromatographie auf Silicagel umfaßt, das aus zwei oder mehreren Lösungsmitteln hergestellt wird, die aus Hexan, Chloroform, Ethylacetat, Benzol und Aceton ausgewählt werden.

**10.** Verfahren zur Herstellung von 4,5-Dihydroxy-2,6,6-trimethyl-2-cyclohepten-1-on beziehungsweise seiner einzelnen Stereoisomere, das die Unterwerfung 4,5-Dihydroxy-2,6,6-trimethylcyclopenta-2-en-1-ons einer Reaktion zur Öffnung der Epoxidringe umfaßt.

**11.** Verfahren nach Anspruch 10, wobei 4,5-Epoxy-2,6,6-trimethyl-2-cyclopenta-2-en-1-on hergestellt wird, indem 2,6,6-Trimethylcyclohepta-2,4-dien-1-on oxidiert wird.

**12.** Verfahren nach den Ansprüchen 10 oder 11, wobei die Reaktion zur Öffnung der Epoxidringe durch die Behandlung von 4,5-Epoxy-2,6,6-trimethylcyclopenta-2-en-1-on mit einer Säure bewirkt wird.

**13.** Verfahren nach den Ansprüchen 11 oder 12, wobei 2,6,6,-trimethylcyclohepta-2,4-dien-1-on oxidiert wird, indem ein Oxidierungsmittel aus organischen oder anorganischen Persäuren und Sauerstoff ausgewählt wird.

**14.** Eine pharmazeutische Zusammensetzung zur Verwendung bei der Behandlung von Magengeschwüren, die 4,5-Dihydroxy-2,6,6-trimethyl-2-cyclohepten-1-on beziehungsweise dessen einzelne Stereoisomere zusammen mit mindestens einem pharmazeutisch zulässigen Bakterienträger oder Arzneistoffträger enthält.

**Patensprüche für folgende Vertragsstaaten : ES, GR**

**1.** Verwendung von 4,5-Dihydroxy-2,6,6-trimethyl-2-cyclohepten-1-on oder seiner einzelnen Stereoisomere zur Herstellung einer Zusammensetzung zur Verwendung bei der Behandlung von Magengeschwüren.

**2.** Verfahren zur Herstellung von 4,5-Dihydroxy-2,6,6-trimethyl-2-cyclohepten-1-on beziehungsweise seinen einzelnen Stereoisomeren, das das Extrahieren derWurzel und des Wurzelstocks des Asiasarum (Asiasari Radix) durch ein organisches Lösungsmittel umfaßt, gefolgt von einer Fraktionierung.

**3.** Verfahren nach Anspruch 2, wobei die Wurzel und der Wurzelstock des Asiasarum (Asiasari Radix) mindestens einmal mit Chloroform extrahiert wird.

**4.** Verfahren nach Anspruch 2 oder Anspruch 3, wobei die Fraktionierung, bei der ein gemischtes Lösungseluens verwendet wird, eine Säulenchromatographie auf Silicagel umfaßt, das aus zwei oder mehreren Lösungsmitteln hergestellt wird, die aus Hexan, Chloroform, Ethylacetat, Benzol und Aceton ausgewählt werden.

**5.** Verfahren zur Herstellung von 4,5-Dihydroxy-2,6,6-trimethyl-2-cyclohepten-1-on beziehungsweise seinen einzelnen Stereoisomeren, das die Unterwerfung 4,5-Dihydroxy-2,6,6-trimethylcyclopenta-2-en-1-ons einer Reaktion zur Öffnung der Epoxidringe umfaßt.

**6.** Verfahren nach Anspruch 5, wobei 4,5-Epoxy-2,6,6-trimethyl-2-cyclopenta-2-en-1-on hergestellt wird, indem 2,6,6-Trimethylcyclohepta-2,4-dien-1-on oxidiert wird.

**7.** Ein Verfahren nach den Ansprüchen 5 oder 6, wobei die Reaktion zur Öffnung der Epoxidringe durch die Behandlung von 4,5-Epoxy-2,6,6-trimethylcyclopenta-2-en-1-on mit einer Säure bewirkt wird.

**8.** Verfahren nach den Ansprüchen 6 oder 7, wobei 2,6,6,-trimethylcyclohepta-2,4-dien-1-on oxidiert wird, indem ein Oxidierungsmittel aus organischen oder anorganischen Persäuren und Sauerstoff ausgewählt wird.

## Revendications

## Revendications pour les Etats contractants suivants : DE, GB, FR, IT, NL, SE, LI, CH, BE, AT, LU, DK

1. Utilisation de la 4,5-dihydroxy-2,6,6-triméthyl-2-cycloheptèn-1-one ou ses stéréo-isomères individuels pour la préparation d'une composition pour l'utilisation dans le traitement des ulcères.

2. 4,5-dihydroxy-2,6,6-triméthyl-2-cycloheptèn-1-one et/ou ses stéréo-isomères individuels.

3. 4,5-dihydroxy-2,6,6-triméthyl-2-cycloheptèn-1-one et/ou ses stéréo-isomères individuels selon la revendication 2, dans une forme sensiblement pure.

4. 4,5-dihydroxy-2,6,6-triméthyl-2-cycloheptèn-1-one et/ou ses stéréo-isomères individuels selon la revendication 2, dans une forme cristalline.

5. 4,5-dihydroxy-2,6,6-triméthyl-2-cycloheptèn-1-one et/ou ses stéréo-isomères individuels selon la revendication 2, essentiellement exempt d'autre produit de la racine et du rhizome d'Asiasarum (Asiasari Radix).

6. 4,5-dihydroxy-2,6,6-triméthyl-2-cycloheptèn-1-one et/ou ses stéréo-isomères individuels selon l'une quelconque des revendications 2 à 5 pour l'utilisation dans le traitement des ulcères.

7. Procédé pour la préparation de la 4,5-dihydroxy-2,6,6-triméthyl-2-cycloheptèn-1-one et/ou ses stéréo-isomères individuels, qui comprend l'extraction de la racine et du rhizome d'Asiasarum (Asiasari Radix) avec un solvant organique, suivie par un fractionnement.

8. Procédé selon la revendication 7, dans lequel la racine et le rhizome d'Asiasarum (Asiasari Radix) sont extraits au moins une fois avec du chloroforme.

9. Procédé selon la revendication 7 ou la revendication 8, dans lequel le fractionnement comprend une chromatographie sur colonne de gel de silice en utilisant un éluant de solvant mixte préparé à partir de deux ou plusieurs solvants choisis parmi l'hexane, le chloroforme, l'acétate d'éthyle, le benzène et l'acétone.

10. Procédé pour la préparation de la 4,5-dihydroxy-2,6,6-triméthyl-2-cycloheptèn-1-one et/ou ses stéréo-isomères individuels, qui consiste à soumettre la 4,5-époxy-2,6,6-triméthylcyclopenta-2-èn-1-one à une réaction d'ouverture du cycle époxyde.

11. Procédé selon la revendication 10, dans lequel la 4,5-époxy-2,6,6-triméthylcyclopenta-2-ène-1-one est préparée par oxydation de la 2,6,6-triméthylcyclohepta-2,4-dièn-1-one.

12. Procédé selon la revendication 10 ou la revendication 11, dans lequel la réaction d'ouverture du cycle époxyde est effectuée par traitement de la 4,5-époxy-2,6,6-triméthylcyclopenta-2-èn-1-one avec un acide.

13. Procédé selon la revendication 11 ou la revendication 12, dans lequel la 2,6,6-triméthylcyclohepta-2,4-dièn-1-oneest oxydée en utilisant un oxydant choisi parmi les peracides organiques ou inorganiques et l'oxygène.

14. Composition pharmaceutique pour l'utilisation dans le traitement des ulcères comprenant de la 4,5-dihydroxy-2,6,6-triméthyl-2-cycloheptèn-1-one et/ou ses stéréo-isomères individuels conjointement avec au moins un support ou un excipient pharmaceutiquement acceptable.

## Revendications pour les Etats contractants suivants : ES, GR

1. Utilisation de la 4,5-dihydroxy-2,6,6-triméthyl-2-cycloheptèn-1-one ou ses stéréo-isomères individuels pour la préparation d'une composition pour l'utilisation dans le traitement des ulcères.

2. Procédé pour la préparation de la 4,5-dihydroxy-2,6,6-triméthyl-2-cycloheptèn-1-one et/ou ses stéréo-isomères individuels, qui comprend l'extraction de la racine et du rhizome de l'Asiasarum (Asiasari Radix) avec un solvant organique, suivie par un fractionnement.

3. Procédé selon la revendication 2, dans lequel la racine et le rhizome de l'Asiasarum (Asiasari Radix) sont extraits au moins une fois avec du chloroforme.

4. Procédé selon la revendication 2 ou la revendication 3, dans lequel le fractionnement comprend une chromatographie sur colonne de gel de silice en utilisant un éluant de solvant mixte préparé à partir de deux ou plusieurs solvants choisis parmi l'hexane, le chloroforme, l'acétate d'éthyle, le benzène et l'acétone.

5. Procédé pour la préparation de la 4,5-dihydroxy-2,6,6-triméthyl-2-cycloheptèn-1-one et/ou ses stéréo-isomères individuels, qui consiste à soumettre la 4,5-époxy-2,6,6-triméthylcyclopenta-2-èn-1-one à une réaction d'ouverture du cycle époxyde.

6. Procédé selon la revendication 5, dans lequel la 4,5-époxy-2,6,6-triméthylcyclopenta-2-èn-1-one est préparée par oxydation de la 2,6,6-triméthylcyclohepta-2,4-dièn-1 one.

7. Procédé selon la revendication 5 ou la revendication 6, dans lequel la réaction d'ouverture du cycle époxyde est effectuée par traitement de la 4,5-époxy-2,6,6-triméthylcyclopenta-2-èn-1-one avec un acide.

8. Procédé selon la revendication 6 ou la revendication 7, dans lequel la 2,6,6-triméthylcyclohepta-2,4-dièn-1-one est oxydée en utilisant un oxydant choisi parmi les peracides organiques ou inorganiques et l'oxygène.